# EUROPEAN PATENT SPECIFICATION

(11) **EP 1 199 983 B1**
(45) Date of publication and mention of the grant of the patent: **12.05.2004**
(21) Application number: 00951148.6
(22) Date of filing: 28.07.2000
(51) Int. Cl.: A61B 5/103

(54) **CHARACTERIZATION OF THE CONTACT DISTRIBUTION BETWEEN TWO ARBITRARY SURFACES USING ELECTRODE ARRAYS**
CHARAKTERISIERUNG DER KONTAKTVERTEILUNG ZWISCHEN ZWEI BELIEBIGEN OBERFLÄCHEN UNTER VERWENDUNG EINER ELEKTRODENANORDNUNG
CARACTERISATION DE LA DISTRIBUTION DU CONTACT ENTRE DEUX SURFACES ARBITRAIRES AU MOYEN D'ENSEMBLES D'ELECTRODES

(30) Priority: 29.07.1999 US 145983 P
(43) Date of publication of application: 02.05.2002
(73) Proprietor: BIO SYNTECH CANADA INC., Laval, Québec H7V 4A7 (CA)
(72) Inventor: BUSCHMANN, Michael, D., Montréal, Québec H4B 2H4 (CA); LEGARE, Anne, Town of Mount Royal, Québec H3P 2E3 (CA); GARON, Martin, Apt. 5, Montréal, Québec H3S 2H6 (CA); SAVARD, Pierre, Ste-Thérèse, Québec J7E 4B3 (CA)
(74) Representative: Evans, Claire
(86) International application number: PCT/CA2000/000885
(87) International publication number: WO 2001/008555

(56) References cited:
- US-A- 5 246 013
- US-A- 5 779 651
- US-A- 5 904 658
- FRANK E H ET AL: "CARTILAGE ELECTROMECHANICS-I. ELECTROKINETIC TRANSDUCTION AND THE EFFECTS OF ELECTROLYTE PH AND IONIC STRENGTH" JOURNAL OF BIOMECHANICS,GB,OXFORD, vol. 20, no. 6, 1987, pages 615-619,621-62, XP002063533

## Description

### BACKGROUND OF THE INVENTION

### (a) Field of the invention

The invention relates to a method for the characterization of the spatial distribution of contact between two arbitrary surfaces and its evolution with time using electrical signals measured by arrays of electrodes present in the contacting surface.

### (b) Description of Prior Art

In the study of material mechanical properties, the characterization of the contact between two surfaces plays a very important role. Several methods exist to characterize the contact and the pressure distribution between two surfaces. A pressure and contact sensor system has been developed for measuring dental occlusion (*US4856993, WO8902727* and *EP0379524*). A flexible tactile sensor was also developed for measuring foot and gaskets pressure distribution (*US5033291, WO9109289* and *EP0457900*). For these systems, two sets of electrodes are separated by a thin pressure-sensitive resistive coating and the change of resistance is measured electronically to determine the timing, force and location of the contact on the surface. Other devices use two or more electrodes placed on the opposing surfaces of a piezoelectric film (*US4216403*, *US5341687* and *US5054323*) or an electroconductive elastomer (*US4163204*) to characterize pressure distributions. A fingerprint sensor using a similar technique has also been developed (*US4394773*).

One common method to measure the contact between two surfaces is by means of a carbon paper material between the two surfaces in contact. The contact can also be characterized with a pressure sensitive copying paper (*US4630079*). These qualitative methods are used as a first indication of the pressure distribution between surfaces but provide no indication of the evolution of the contact with time.

Methods have also been developed to detect the contact between two surfaces without studying the evolution of the contact with time or measuring the pressure distribution. For example, methods were developed to determine the contact between biomedical instruments and tissues, such as a catheter and a tissue during ablation. In this case, the voltage between an electrode on the catheter and another electrode on the patient is measured (*WO9843547A2*). Other techniques use a measurement of the ratio of the change of the total contact impedance of the electrode (*WO9909899A1*) or a measurement of the phase angle of current flow in two different portions of an electrode (*WO9844856*) to detect the loss of contact between a biomedical electrode and skin.

Some techniques are also used to study the topography of a surface, like the precision small scale force sensor which is based on the resultant attractive and/or repulsive forces between the probe and the surface that produce deformation of a strain gauge material resulting in a change of its electrical properties (*US5092163*). In this case, the electrode is not in contact with the surface.

The US Patent No: 5,779,651 discloses a medical apparatus for the early detection and the diagnosis of cartilage degeneration and a method of using such apparatus. However, this invention provides no information about the surface of contact between the device tip and the material being characterized, or on the temporal evolution of this surface of contact between the indenting surface of the device and the material to be characterized. This is a critical point since in the absence of accurate knowledge of the contact area and how it changes over time, no deduction of material properties can be made.

It would thus be highly desirable to be provided with a method that would allow a direct and precise characterization of the time of contact between two surfaces, and thus indirectly of the deformation applied and the mechanical response of the surface.

### SUMMARY OF THE INVENTION

One aim of the present invention is to provide a method for determining precisely the time of contact between two surfaces using multiple electrical signals from electrodes exposed on the contacting surface.

Another aim of this invention is to use the electrical signals indicating time evolution of contact distribution to estimate the amplitude and velocity of the displacement applied to a tested material over time and the angular orientation of the electrodes containing surface during contact and compression.

Another aim of this invention is to allow characterization of the mechanical response of a material via the nature and temporal evolution of the contact between two surfaces.

Another aim of this invention is to provide various surface configurations with a multidimensional array of electrodes to evaluate precisely the contact distributions, the applied displacement and the mechanical response of a material.

In accordance with the present invention, there is provided a new in vitro method to characterize the evolution of the contact distribution between two surfaces with time using arrays of electrodes present in the contacting surface. By placing electrodes at defined locations on an arbitrary surface and measuring the electrical signal generated with time during compression of a second arbitrary surface, the evolution of the contact can be precisely characterized and thus essential information on the mechanical response of the material is provided. This method differs from the existing methods in two main aspects. First, instead of measuring a resistance with two electrodes on the opposite sides of a pressure sensitive coating, arrays of electrodes are used on a contacting surface and electrical potentials during compression of a second arbitrary surface are measured. The second difference is the type of measurement. Instead of measuring a pressure, differential or absolute electrical potentials generated during contact and compression of the tested material are measured. Such information can be used to estimate the displacement applied to the material as well as the angular orientation of the surface containing the electrodes relative to the material during contact and compression. In addition, the mechanical response of the material is directly indicated by its ability to conform to the surface containing the electrodes.

Generation of force between two contacting surfaces depends strongly on the time evolution of the contact, and knowledge of the contact evolution with time is necessary to interpret force signals. Another example where the contact information is important is in the measurement of compression-generated electric fields. A typical example is when a probe containing microelectrodes comes into contact with and compresses a substance to be characterized via the electrical potentials generated at the exposed microelectrodes. When such compression is applied manually, thus lacking precise information on the type of displacement imposed, the distribution of contact with time can provide information on the type of displacement applied, even when it is poorly controlled as in the case of a manual application. Such information could be of the form of the displacement or velocity of the probe over time and its angular orientation with respect to the tested substance. Precise measurement of contact distribution with time could also lead to a new type of materials analysis which in its most general form could be thought of as characterizing a "surface compliance".

### BRIEF DESCRIPTION OF THE DRAWINGS

Having thus generally describe the nature of the invention, references will now be made to the accompanying drawings, showing by way of illustration a preferred embodiment thereof, and wherein:
Figs. 1A, 1B and 1C illustrate different surface geometry with arrays of electrodes used with the method of the present invention;
Fig. 2 illustrates typical differential electrical signals and load measured with the method of the present invention when a semi conical surface (Fig. 1B) containing electrodes is brought into contact with a specimen of articular cartilage placed in saline solution;
Fig. 3 illustrates typical differential electrical signals and load measured with the method of the present invention when a semi conical surface (Fig. 1B) containing electrodes is brought into contact by manual means with a specimen of articular cartilage placed in saline solution ;
Fig. 4 illustrates typical differential electrical signals and load measured with the method of the present invention when a semi conical surface (Fig. 1B) containing electrodes is brought into contact with an arbitrary non-biological elastomeric material, in this case an eraser;
Fig. 5A illustrates typical absolute electrical signals and load measured with the method of the present invention when a semi spherical surface (Fig. 1C) containing electrodes is brought into contact with a specimen of articular cartilage placed in saline solution;
Fig. 5B illustrates the time difference between the start of signals, thereby demonstrating the temporal evolution of contact between the surface containing electrodes and the cartilage surface as well as the angular orientation of the surface containing electrodes, by superposing the absolute electrical signals of Fig. 5A;
Fig. 6A illustrates typical absolute electrical signals and load measured with the method of the present invention when a semi spherical surface (Fig. 1C) containing electrodes is brought into contact by manual means with a specimen of articular cartilage placed in saline solution;
Fig. 6B illustrates the time difference between the start of signals, thereby demonstrating the temporal evolution of contact between the surface containing the electrodes and the cartilage surface as well as the angular orientation of the surface containing the electrodes, by superposing the electrical signals of Fig. 6A;
Fig. 7 illustrates typical absolute electrical signals and load measured with the method of the present invention when a semi spherical surface (Fig. 1C) is brought into contact with an elastomeric material;
Fig. 8 illustrates typical absolute electrical signals and load measured with the method of the present invention when a semi spherical surface (Fig. 1C) containing the electrodes is first brought into contact with a specimen of articular cartilage placed in saline solution and then released from the surface by movement in the opposite direction; and
Fig. 9 illustrates typical differential electrical signals and load measured with the method of the present invention during compression with a semi conical surface (Fig. 1B) containing electrodes of healthy and degraded cartilage explants placed in saline solution.

### DETAILED DESCRIPTION OF THE INVENTION

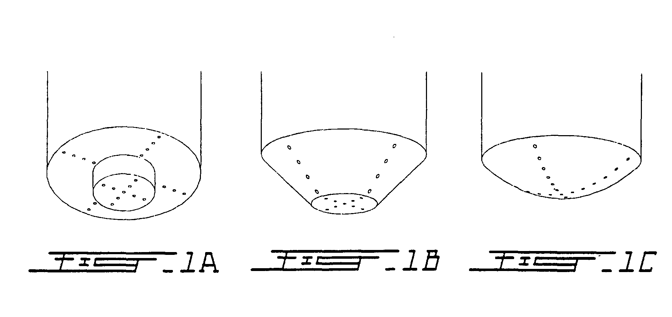

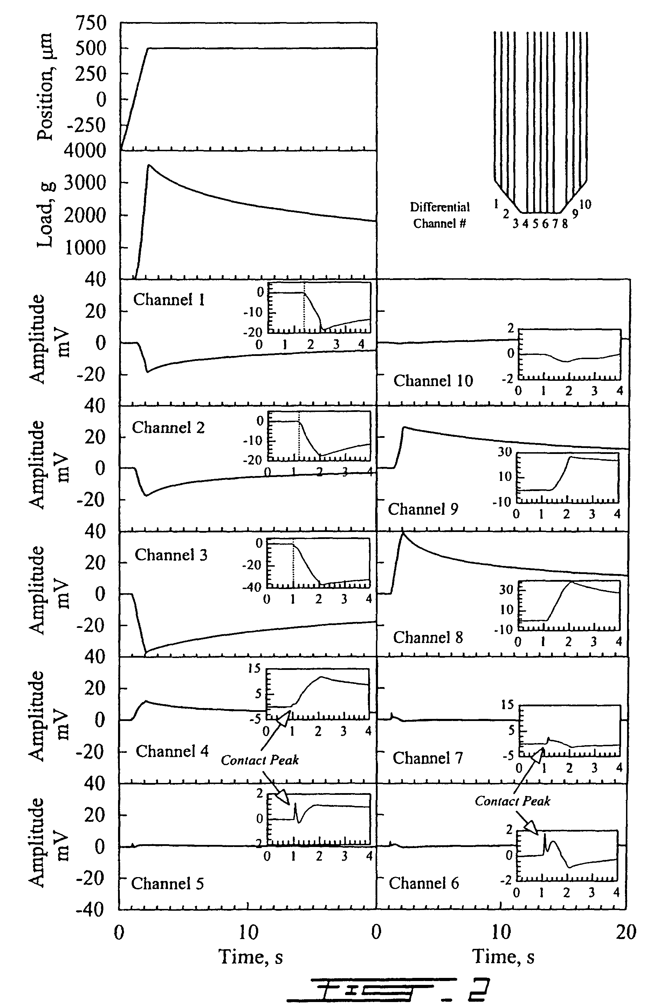

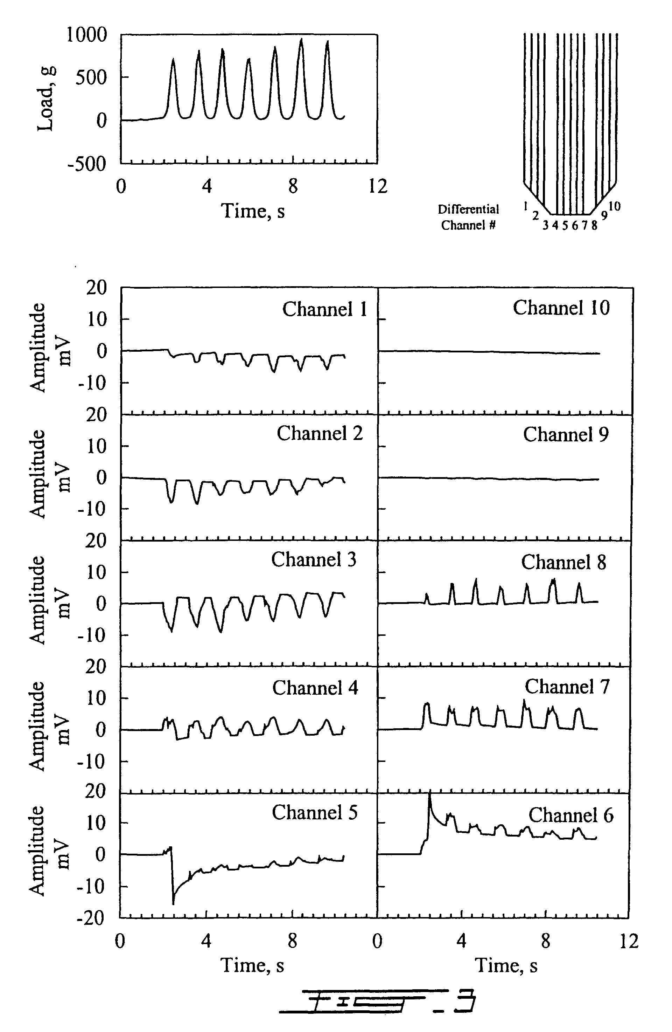

The contact between two arbitrary surfaces can be characterized by the electrical signals measured by electrodes located on one of the surfaces. Furthermore, the surface containing the electrodes can put into contact with any material surface. Figs. 1A to 1C show particular examples of a wide range of surface geometries and configurations of electrodes that can be used to characterize the contact between two surfaces. The electrode geometry, size, spacing and material type can vary depending on the application.

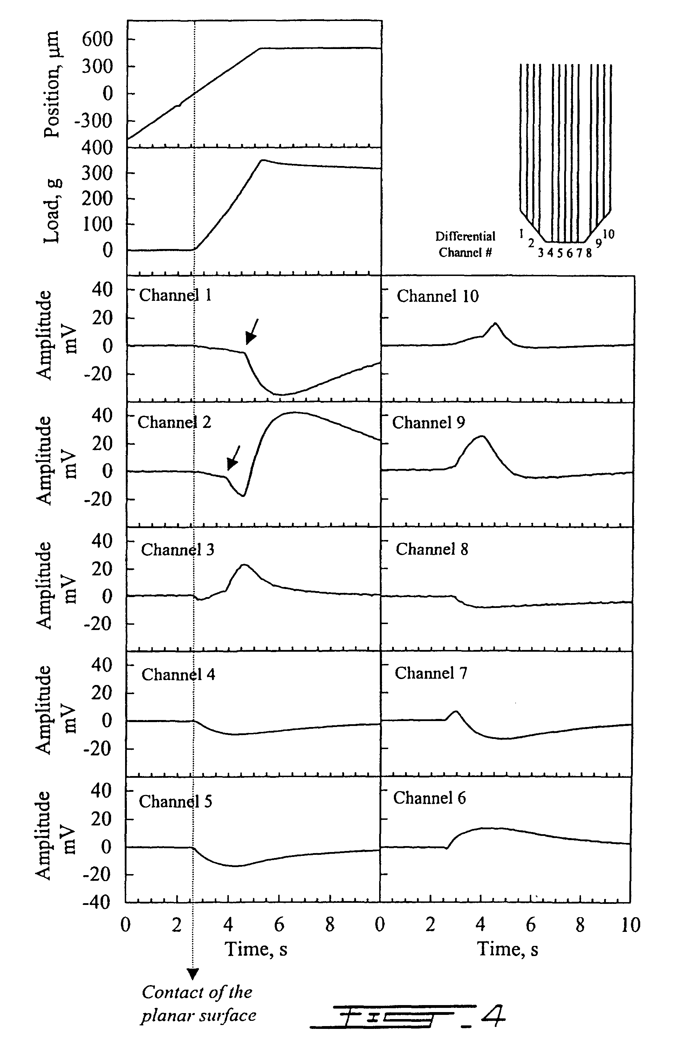

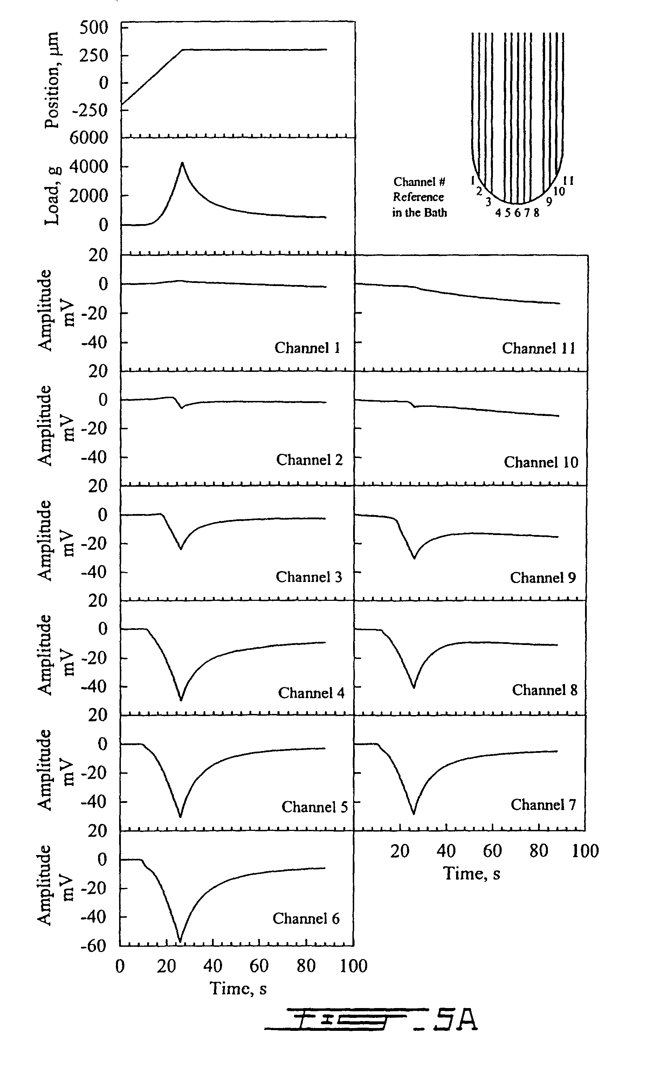

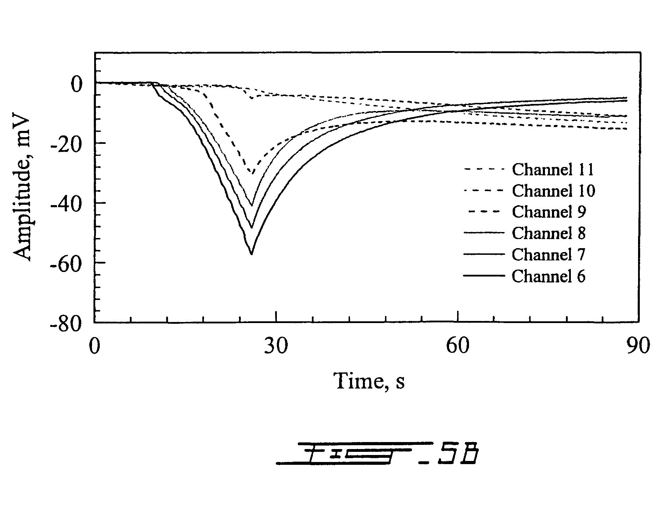

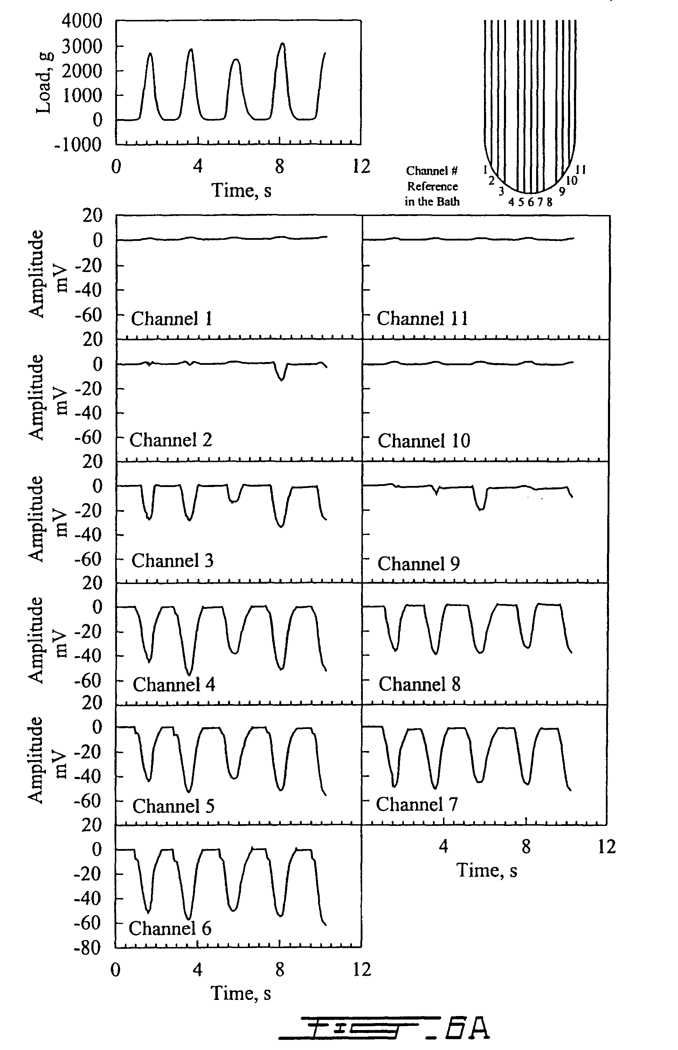

During the contact of two arbitrary surfaces, electrical signals can be measured between two adjacent electrodes (differential measurements) or between each electrode and a common reference (absolute measurements). The common reference can be a reference electrode placed in the testing bath and connected to ground or a particular electrode of the array contacting or not the second surface. Noise content of the electrical signals can be significantly reduced in the case of differential measurements or absolute measurements with another electrode of the array used as reference. Figs. 2, 3, 4 and 9 illustrate examples of differential measurements. Figs. 5A, 5B, 6A, 6B, 7 and 8 illustrate examples of absolute measurements relative to a reference electrode placed in the testing bath and connected to ground.

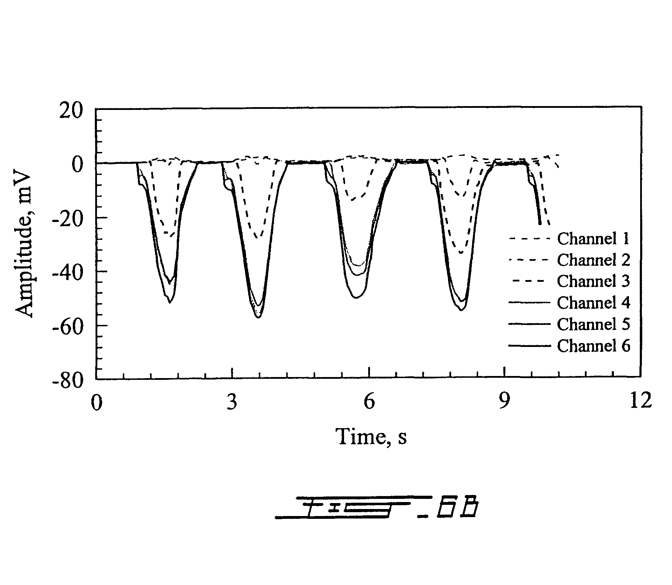

Electrical signals were measured with a linear array of 11 platinum electrodes of 50 µm diameter spaced by 300 µm except for two pairs of electrodes which were spaced apart by 600 µm (electrodes 3 and 4 and electrodes 8 and 9). Figs. 2, 4, 5A, 5B, 7, 8 and 9 show electrical signals measured during a stress relaxation test. To control precisely the amplitude and velocity of the contact and the deformation and to measure the force applied to the surface, a mechanical testing system was used. The surface containing the electrodes or the post was connected to a load cell, the tissue was fixed in a testing chamber filled with saline solution and mounted on an actuator platform and a displacement was applied by the actuator. During a typical stress relaxation test, the post containing the electrodes was first brought into contact with the tissue (position 0 on the position versus time graph). Then, the two surfaces were separated by a known distance and a compression step was applied with a fixed velocity. Figs. 3, 6A and 6B present electrical signals measured during a manual compression of the material with the post containing the electrodes connected to the load cell.

Fig. 2 shows the differential electrical signals measured during a stress relaxation test on a specimen of articular cartilage placed in saline solution. Articular cartilage is a biological tissue composed of fixed negative charges. During compression of cartilage, there is displacement of mobile positive ions in the fluid relative to fixed negative charges, producing electrical signals or streaming potentials. During this stress relaxation test, the cartilage surface was compressed by 500 µm in 1 second with a semi conical post containing the electrodes. A schematic drawing of the semi conical surface show the position of each differential channel which represents the potential difference between adjacent electrodes (10 channels for 11 electrodes). First, an increase of the load is observed when the two surfaces come into contact (position 0 on the position versus time graph). The load is maximal at the end of the displacement and starts to decrease during the relaxation of the tissue to reach equilibrium. Similar behaviors were observed for the electrical signals measured for each channel. It is also noted that the four differential channels on the planar surface (channels 4, 5, 6 and 7) have a lower amplitude than channels located on the conical surfaces and are characterized by a contact peak at 1 second. Channels located on the conical surfaces are characterized by higher potential amplitudes because of the higher pressure difference between adjacent electrodes. In fact, during compression, the planar surface of the post is touching the tissue first applying the higher deformation, so that the pressure is maximal on that surface. However, even if the pressure is maximal, the pressure difference between two adjacent electrodes located on the planar surface is small because the pressure is uniform. For the electrodes located in the conical region, the pressure is decreasing, being minimal at the periphery, so that pressure differences are higher (Soulhat J et al., *J Biomech Eng* **121**:340, 1999). The time at which each electrode pair touches the tissue can be deduced. For channels 3, 2 and 1, the potential starts to increase at 1, 1.2 and 1.4 seconds respectively. Similar results are observed for the symmetric channels 8, 9 and 10. Channels 3 and 8 are characterized by higher amplitudes because electrode pairs are spaced by 600 µm instead of 300 µm. Furthermore, symmetric channels located on each side of the planar surface have opposite signs (channels 1, 2 and 3 relative to channels 10, 9 and 8) because of the opposite direction of the fluid flow during compression. In fact, the pressure applied to the tissue with the semi conical post is minimal at the periphery of the post, increases to a maximal value at the center and decreases again from the center to the periphery. Since potentials are measure with a linear array of electrodes in one direction, symmetric channels on each side of the planar surface have opposite signs.

Fig. 3 shows another example of the differential electrical signals measured when a semi conical surface is brought into contact with a specimen of cartilage placed in saline solution by manual means. As seen in Fig. 2, the shape of the measured electrical signals is similar to the mechanical load response of the tissue. Once again, channels located on each side of the planar surface have opposite sign. In this particular example, the amplitude of channels 9 and 10 are very low, because of the angular orientation of the post during the manual compression. The results of Figs. 2 and 3 show that complementary information can be obtained from the electrical signals and the load during contact and compression.

Fig. 4 illustrates an example of the differential electrical signals measured when a semi conical post with electrodes is brought into contact with an elastomeric material. This test was done to demonstrate that the contact distribution could be characterized on materials other than biological tissues. Since an elastomeric material has no fixed ionized charged groups, , there is no displacement of mobile ions relative to fixed charges during compression, in contrast to biological tissues such as cartilage. This test was performed dry without immersion in saline solution. Furthermore, the electrical signal offset was eliminated by high pass filtering. Results of Fig. 4 show potentials starting to increase later for channel 2 and 1 which are located on the conical part of the surface containing the electrodes, compared to channels 4 or 5 which are on the planar surface. The arrows on channels 1 and 2 indicate the time of contact of one of the two electrodes constituting the adjacent electrodes pair of the channel. These results demonstrate the feasibility of characterizing the contact distribution using the method of the present invention on any sample material.

Fig. 5A presents the absolute electrical signals measured during a stress relaxation test on a specimen of articular cartilage in saline solution. During this test, the cartilage surface was compressed by 300 µm in 15 seconds with a semi spherical post containing the electrodes. A schematic drawing of the semi spherical surface show the position of each absolute channel which represents the potential difference between each electrode and a reference electrode placed in the testing bath and connected to ground (11 channels for 11 electrodes). As in Fig. 2, the load starts to increase at contact to a maximal value and then relaxes to reach equilibrium. The shape of the electrical signals is similar to the mechanical load response of the tissue. Channel 6 (potential difference between the electrode 6 and the reference electrode in the bath connected to ground) is characterized by the highest amplitude. In fact, electrode 6 is the first electrode to touch the tissue. Electrode 6 also contacts the cartilage where the largest deformation is applied and the pressure is maximal. The amplitude of other channels decreases symmetrically from the center (channel 6) to the periphery (channel 1 or 11). Electrical signals of all channels are negative and in phase because signals were measured relative to a reference electrode connected to ground and cartilage is composed of fixed negative charges. In fact, during compression, positive mobile ions are displaced relative to fixed negative charges in the direction of the reference electrode and the measured electrical signal is negative.

Fig. 5B shows a superposition of the electrical signals measured on one of the two symmetric sides of the semi spherical post. Once again, an increase of the signals amplitude from the periphery (channel 11) to the center (channel 6) can be observed. The time delay between signals can also clearly be seen. The time delay is the time difference between the start of two signals, i.e. when they begin to differ from zero or become negative. By knowing the exact geometry of the semi spherical surface and the position of each electrode as well as the time delay between the start of each signal, the temporal evolution of contact and information concerning the compression amplitude and velocity and the angular orientation of the surface containing the electrodes relative to the other surface during compression can be precisely deduced. In the case where the surface containing the electrodes is not perpendicular to the other surface, potential signals are in advance (shorter time delay) and amplitude are higher for channels on the region of the semi spherical surface that touches the tissue first.

Fig. 6A presents the absolute electrical signals measured during manual compression with a semi spherical post of a specimen of cartilage placed in saline solution. Once again, the shape of the electrical signals is almost identical to the mechanical load response of the cartilage. The amplitude is higher at the center (channel 6) and decreases symmetrically from the center to the periphery.

Fig. 6B shows a superposition of the absolute electrical signals measured on one side of the semi spherical post. Once again, an increase of the signals amplitude from the periphery (channel 1) to the center (channel 6) is observed. The time delay between the start of electrical signals can also be observed. Time delays shown on Fig. 6B are less visible than those of Fig. 5B, because manual compression was more rapid. However, amplitude, velocity and angular orientation of the post during compression can be deduced here by using higher data acquisition rates.

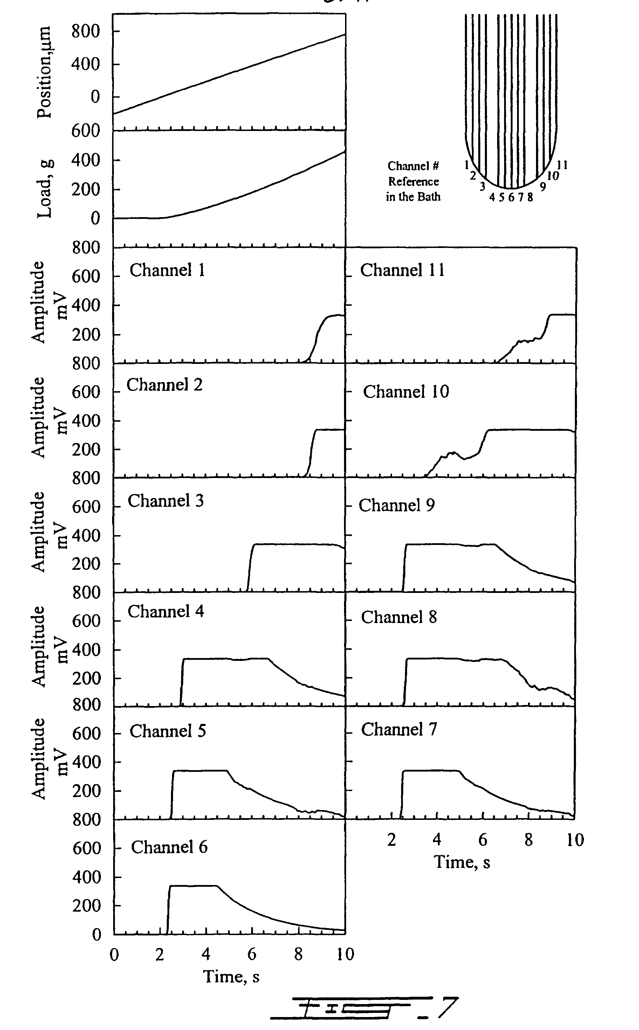

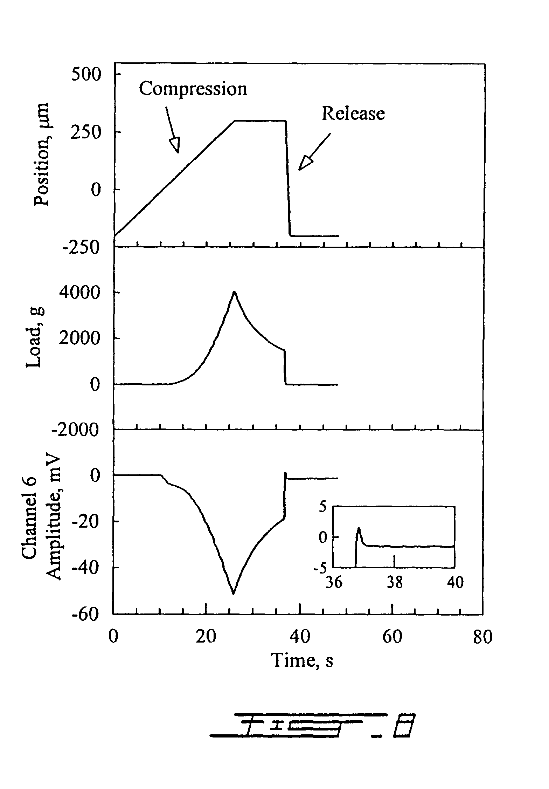

Fig. 7 presents the absolute electrical signals measured during compression of an elastomeric material with a semi spherical post. This test was performed dry without immersion in saline solution. Furthermore, the electrical signal offset was eliminated by high pass filtering. First, there is saturation of the electrical amplification system just after contact between the post and the elastomeric material. However, an increase of the time delay in the electrical signals from the center to the periphery can clearly be seen. Fig. 8 shows the absolute electrical signal of channel 6 measured during the compression of cartilage with a semi spherical post. In this case, the post was rapidly released after the compression. Additional electrical information is obtained during release of the surface. Furthermore, a small upward peak is seen during release in channel 6 at a time of 37 seconds. Once again, the form of electrical signal is similar to the mechanical load response on the tissue during the compression.

As illustrated, absolute electrical signals (Figs. 5A, 5B, 6A, 6B, 7 and 8) may more easily reflect the evolution of contact over time than differential electrical signals (Figs. 2, 3, 4 and 9). In fact, the contact was less evident for differential measurements since the electrical signals were measured between two adjacent electrodes located at different position on the surface and touching the tissue at different times. However, in the two cases, the time of contact between the two surfaces as well as the evolution of the contact with time can be precisely determined. The evolution of the contact with time gives information about the ability of the material to conform to the surface containing the electrodes. Furthermore, with the exact position of the electrode on the surface, the amplitude, the velocity and the angular orientation of the surface containing the electrodes during contact and compression can easily be deduced.

Depending on the type of material to be tested, measured electrical signals can be passed through a conditioning preamplifier, an amplifier and filters before being acquired by a computer. In all cases, the contact impedance between the electrodes and the material must be negligible compared to the input impedance of the preamplifier. Furthermore, the bias current must be minimal to avoid electrode polarization. To minimize the contact impedance, electrodes can be coated with chloroplatinic solution in an ultrasound bath.

The present invention will be more readily understood by referring to the following example, which is given to illustrate the invention rather than to limit its scope.

### EXAMPLE 1

Figs. 2, 3, 5A, 5B, 6A, 6B and 8 show that the electrical signals measured on the cartilage surface are directly related to the mechanical properties of the tissue. In fact, similar information can be obtained from the shape of the mechanical load response and the measured potentials during contact and compression of articular cartilage. From a mechanical point of view, articular cartilage is a material composed of solid and fluid phases. The fluid phase (water) is located in the microscopic pores of the tissue. The size of the pores is around 5 nm. When a compression is applied to the cartilage, interstitial fluid flows through the porous matrix generating large pressure forces which resist compression and water loss. Cartilage therefore demonstrates a characteristic viscoelastic behavior so that under load or deformation, articular cartilage possesses a time dependent mechanical behavior. Streaming potentials are directly related to the pressure generated during compression of the cartilage. Furthermore, the fluid velocity during compression is related to the streaming potential gradient. For example, in figure 5A, we have observed that the channel 6 (streaming potential measured between electrode 6 and a reference electrode connected to ground) was characterized by the higher streaming potential amplitude which is directly related to the maximal pressure applied by the semi spherical post.

By integrating the multidimensional electrodes array into the tip of an arthroscopic probe, streaming potentials can be measured directly on the surface of cartilage inside an articulation . However, to determine precisely the electromechanical properties of the cartilage, the compression amplitude or deformation and velocity applied to the cartilage is needed. By choosing the geometry of the surface containing the electrodes and the distance between the electrodes and by measuring precisely the time of contact of each electrode, the displacement applied to cartilage (precise estimation depends on knowledge of the mechanical response of the tested material) as well as the angular orientation of the surface containing the electrodes during compression can be estimated. Such information can be useful in the context of the medical apparatus for the diagnosis of cartilage degeneration via spatial mapping of compression-induced electrical potentials.

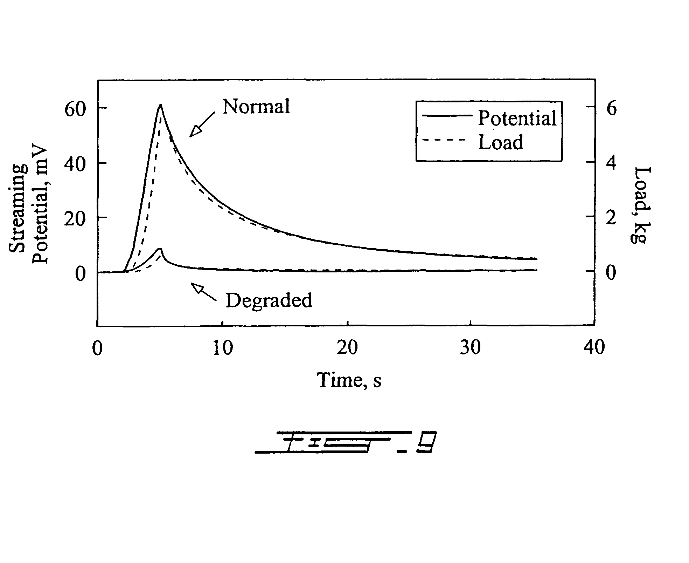

The extracellular matrix of cartilage is primarily composed of fixed negatively charged molecules called proteoglycans entrapped in a collagen network. During the compression of cartilage, there is displacement of positive mobile ions in the fluid relative to the fixed negatively charged proteoglycans, producing streaming potentials. Osteoarthritis is a degeneration of the cartilage reflected by the loss of proteoglycans and the loss of integrity of the collagen network. The streaming potentials measured on the cartilage surface are directly related to the proteoglycan content and collagen network integrity, so that osteoarthritis is reflected by a diminution of the streaming potentials amplitude. The arthroscopic probe can thus be used to determine precisely the state of health or degeneration of a tissue and to test methods of regeneration and other tissue treatments. Fig. 9 illustrates an example of differential potentials (channel 3) measured on normal and degraded cartilage explants during compression with a semi conical post. In this case, a healthy cartilage explant was treated with a biochemical degradation agent to induce proteoglycan loss and collagen network breakdown. Streaming potentials were measured periodically to study the evolution of the degradation. As can be seen, streaming potentials are particularly sensitive to degradation. Furthermore, streaming potentials and load have almost identical behavior for normal and degraded cartilage.

During an arthroscopic test, the orthopedist will apply manually a small compression to the cartilage. This procedure will be repeated several times to ensure reproduction of the results. The orthopedist can not necessarily precisely control the displacement applied to cartilage. Furthermore, the surface containing the electrodes will never be perfectly parallel to cartilage surface. With a planar, semi conical or semi spherical surface and a multidimensional array of electrodes, electrical signals can be analyzed to determine precisely the contact time of each electrode with the tissue, and thereby to indicate the applied relative displacement and the angular orientation of the two surfaces. The parallelism problem can be overcome using a spherical surface containing the electrodes, and signal analysis allowing for arbitrary orientation of the surface containing the electrodes.

## Claims

1. An in vitro method of characterisation of the temporal and spatial evolution of contact between two arbitrary surfaces using electrode arrays, said method comprising the steps of:
a) applying a probe surface containing at least one array of electrodes against a specimen surface causing said electrodes to generate signals in response to contacting the specimen surface;
b) obtaining the signals from said electrodes; and
c) analysing said signals indicating time evolution of contact distribution to estimate amplitude and velocity of a displacement applied on said specimen surface over time and to estimate angular orientation of said probe surface.

2. The method of claim 1, wherein said probe surface is non-planar.

3. The method of claim 1, or 2, wherein the array of electrodes comprises a plurality of arrays extending in different dimensions.

4. The method of claim 1, 2 or 3, wherein the array of electrodes is positioned on the surface containing the array of electrodes to form lines, circles or curvilinear forms.

5. The method of claim 1, 2, 3 or 4, wherein the geometry of the specimen surface and of the surface containing the array of electrodes have a geometry selected from the group consisting of complete or partial planar surface, revolutionary surface, irregular surface or a combination of two or more surfaces.

6. The method of claim 1, 2, 3, 4, or 5, wherein the array of electrodes comprises at least three electrodes, said at least three electrodes defining two dimensions.

7. The method of claim 1, 2, 3, 4, 5 or 6, wherein an electrical potential is measured in a differential way as the potential difference between adjacent electrodes.

8. The method of claim 1, 2, 3, 4, 5 or 6, wherein an electrical potential is measured in an absolute way relative to a fixed electrode of the array or a reference electrode placed in a testing bath and connected to the ground.

9. The method of claim 1, 2, 3, 4, 5, 6, 7 or 8, wherein the signals are measured by capacitive coupling with contactless electrodes having stable noise characteristics, absence of any corrosive effects and insulation properties.

10. Use of the method of any one of claims 1 to 9 for indicating at least one of the amplitude and velocity of the displacement and the orientation the surface containing said at least one array of electrodes with respect to the specimen surface.

11. Use of the method of any one of claims 1 to 9 for characterising the mechanical response or the "surface compliance" of a material.

12. An in vitro method for detecting the condition of a tissue including composition, structure, function, state of health or disease of the tissue using electrical potentials induced by compression of the tested tissue against a probe surface containing exposed electrodes, said method comprising the steps of:
a) characterising temporal and spatial evolution of contact between two arbitrary surfaces using at least one array of electrodes, said characterising temporal and spatial evolution of contact between two arbitrary surfaces being in accordance with the method defined in any one of claims 1 to 9; and
b) interpreting said temporal and spatial evolution of contact between said two arbitrary surfaces to determine the condition of the tissue.

13. The method of claim 12, wherein said probe surface is non-planar.

14. The method of claim 12 or 13, wherein the tissue is cartilage.

15. An apparatus for characterising the temporal and spatial evolution of contact between two arbitrary surfaces, comprising:
a probe having a tip;
at least one array of electrodes on the tip for generating signals in response to contact between a probe surface and a specimen surface;
signal acquisition and processing means for characterisation of the temporal and spatial evolution of contact between two arbitrary surfaces, said characterisation comprising analysing said signals to estimate amplitude and velocity of a displacement applied on said specimen surface over time and to estimate angular orientation of said probe surface.

16. The apparatus of claim 15, wherein the electrodes are made of metal selected from the group consisting of noble metal, silver, gold, palladium, platinum, rhodium, ruthenium, iridium, osmium, copper, aluminum, and tungsten, or an alloy of several metals selected from silver/silver chloride, stainless steel, and platinum/iridium or any combination of the metal mentioned above.

17. The apparatus of claim 15 or 16, wherein the electrodes are made of metal wires.

18. The apparatus of claim 15, 16 or 17, wherein the electrodes have a diameter from sub-micron to millimeter ranges.

19. The apparatus of claim 15, 16, 17 or 18, wherein the processing means are in closed proximity to the electrodes for reducing noise pick up and microphonic effects.

20. The apparatus of claim 15, 16, 17, 18 or 19, wherein the surface of electrodes is treated to reduce electrical contact impedance.

21. The apparatus of claim 15, 16, 17, 18, 19 or 20, wherein the processing means comprises a voltage follower having a high input impedance and a low bias current to maximise the common-mode rejection ratio for measured voltages.

22. An apparatus for diagnosing a tissue condition, comprising:
a probe having a tip;
at least one array of electrodes on the tip for generating signals in response to contact between a probe surface and a specimen surface;
signal acquisition and processing means for detecting the condition of a tissue including composition, structure, function, state of health or disease of the tissue using electrical potentials induced by compression of the tested tissue against a probe surface containing exposed electrodes, said detecting the condition of a tissue comprising analysing said signals indicating time evolution of contact distribution to estimate amplitude and velocity of a displacement applied on said specimen surface over time and to estimate angular orientation of said probe surface, and interpreting said amplitude and velocity of said displacement and said angular orientation of said probe surface to determine the condition of the tissue.

23. The apparatus of claim 22, wherein the electrodes are made of metal selected from the group consisting of noble metal, silver, gold, palladium, platinum, rhodium, ruthenium, iridium, osmium, copper, aluminum, and tungsten, or an alloy of several metals selected from silver/silver chloride, stainless steel, and platinum/iridium or any combination of the metal mentioned above.

24. The apparatus of claim 22 or 23, wherein the electrodes are made of metal wires.

25. The apparatus of claim 22, 23 or 24, wherein the electrodes have a diameter from sub-micron to millimeter ranges.

26. The apparatus of claim 22, 23, 24 or 25, wherein the processing means are in close proximity to the electrodes for reducing noise pick up and microphonic effects.

27. The apparatus of claim 22, 23, 24, 25 or 26, wherein the surface of electrodes is treated to reduce electrical contact impedance.

28. The apparatus of claim 22, 23, 24, 25, 26 or 27, wherein the processing means comprises a voltage follower having a high input impedance and a low bias current to maximize the common-mode rejection ratio for measured voltages.

## Patentansprüche

1. In-Vitro-Verfahren zur Charakterisierung des zeitlichen und räumlichen Verlaufs des Kontakt zwischen zwei beliebigen Flächen unter Verwendung von Elektroden-Arrays, wobei das Verfahren die Schritte aufweist:
a) Anlegen einer Sondenfläche, die zumindest ein Array von Elektroden enthält, an einer Probenfläche, um die Elektroden zu veranlassen, Signale in Reaktion auf das Kontaktieren der groben Fläche zu erzeugen;
b) Gewinnen der Signale von den Elektroden;
c) Analysieren der Signale, welche den zeitlichen Verlauf der Kontaktverteilung anzeigen zur Ermittlung der Amplitude und Geschwindigkeit einer Verschiebung, die auf die Probenflächc im Lauf der Zeit ausgeübt wird, und zum Ermitteln der winkelmäßigen Ausrichtung der Sondenfläche.

2. Verfahren nach Anspruch 1, wobei die Sondenfläche nicht planar ist .

3. Verfahren nach einem der Ansprüche 1 oder 2, wobei das Array von Elektroden mehrere Arrays umfasst, die sich in unterschiedlichen Dimensionen erstrecken.

4. Verfahren nach Anspruch 1, 2 oder 3, wobei die Gruppierung von Elektroden auf derjenigen Fläche zu liegen kommt, welche die Gruppierung von Elektroden enthält, um Linien, Kreise oder krummlinige Formen zu bilden.

5. Verfahren nach Anspruch 1, 2, 3 oder 4, wobei die Geometrie der Probenfläche und der das Array von Elektroden enthaltenden Fläche eine Geometrie aufweist, die aus der Gruppe ausgewählt ist, die aus einer vollständig oder teilweise planaren Fläche, einer Rotationsfläche, einer unregelmäßigen Fläche bzw. einer Kombination aus zwei oder mehr Flächen besteht.

6. Verfahren nach Anspruch 1, 2, 3, 4 oder 5, wobei die Gruppierung von Elektroden zumindest drei Elektroden umfasst, wobei die zumindest drei Elektroden zwei Dimensionen festlegen.

7. Verfahren nach Anspruch 1, 2, 3, 4, 5 oder 6, wobei ein elektrisches Potential als Potentialdifferenz zwischen benachbarten Elektroden differentiell gemessen wird.

8. Verfahren nach Anspruch 1, 2, 3, 4, 5 oder 6, wobei ein elektrisches Potential relativ zu einer stationären Elektrode des Array oder einer Referenzelektrode absolut gemessen wird, die in einem Testbad zu liegen kommt und mit Masse verbunden ist.

9. Verfahren nach Anspruch 1, 2, 3, 4, 5, 6, 7 oder 8, wobei die Signale durch kapazitive Kopplung mit kontaktfreien Elektroden gemessen werden, die stabile Rauschcharakteristik, keinerlei korrosive Wirkung und Isolationseigenschaften aufweisen.

10. Verwendung des Verfahrens nach einem der Ansprüche 1 bis 9 zum Anzeigen von zumindest entweder der Amplitude oder der Geschwindigkeit der Verschiebung und der Ausrichtung der Fläche, die zumindest eine Reihe von Elektroden enthält, in Bezug auf die Probenfläche.

11. Verwendung des Verfahrens nach einem Ansprüche 1 bis 9 zum Charakterisieren der mechanischen Reaktion bzw. der natürlichen "Flächennachgiebigkeit" eines Materials.

12. In-Vitro-Verfahren zum Ermitteln des Zustands eines Gewebes, einschließlich Zusammensetzung, Struktur, Funktion, Gesundheitszustand bzw. Erkrankung des Gewebes unter Verwendung elektrischer Potentiale, die induziert werden durch drücken des getesteten Gewebes auf eine Sondenfläche, die freiliegende Elektroden enthält, wobei das Verfahren die Schritte aufweist:
a) Charakterisieren des zeitlichen und räumlichen Verlaufs des Kontakts zwischen zwei beliebigen Flächen unter Verwendung von zumindest einem Array von Elektroden, wobei das Charakterisieren des zeitlichen und räumlichen Verlaufs des Kontakts zwischen zwei beliebigen Flächen in Übereinstimmung mit dem Verfahren erfolgt, das in einem der Ansprüche 1 bis festgelegt ist, und
b) Interpretieren des zeitlichen und räumlichen Verlaufs des Kontakts zwischen den beiden beliebigen Flächen zu ermitteln des Zustands des Gewebes.

13. Verfahren nach Anspruch 12, wobei die Sondenfläche nicht planar ist.

14. Verfahren nach Anspruch 12 oder 13, wobei. das Gewebe ein Knorpel ist.

15. Vorrichtung zum Charakterisieren des zeitlichen und räumlichen Verlaufs des Kontakts zwischen zwei beliebigen Flächen aufweisend:
Eine Sonde mit einer Spitze;
zumindest ein Array von Elektroden auf der Spitze zum Erzeugen von Signalen in Reaktion auf einen Kontakt zwischen einer Sondenfläche und einer Probenfläche;
eine Signalerfassungs- und Verarbeitungseinrichtung zum Charakterisieren des zeitlichen und räumlichen Verlaufs des Kontakts zwischen zwei beliebigen Flächen, wobei die Charakterisierung das Analysieren des Signals umfasst, um die Amplitude und Geschwindigkeit einer Verschiebung zu ermitteln, die auf die Probenfläche im Verlauf der Zeit ausgeübt wird, und zum Ermitteln der winkelmäßigen Ausrichtung der Sondenfläche.

16. Vorrichtung nach Anspruch 15, wobei die Elektroden aus Metall hergestellt sind, das aus der Gruppe ausgewählt ist, die aus Edelmetall, Silber, Gold, Palladium, Platin, Rhodium, Ruthenium, Iridium, Osmium, Kupfer, Aluminium und Wolfram oder einer Legierung aus mehreren Metallen besteht, die aus Silber/Silberchlorid, Edelstahl und Platin/Iridium oder einer beliebigen Kombination der vorstehend genannten Metalle ausgewählt sind.

17. Vorrichtung nach Anspruch 15 oder 16, wobei die Elektroden aus Metalldrähten hergestellt sind.

18. Verfahren nach Anspruch 15, 16 oder 17, wobei die Elektroden einen Durchmesser aufweisen der in einem Bereich von Sub-Mikrometer bis Millimeter liegt.

19. Vorrichtung nach Anspruch 15, 16, 17 oder 18, wobei die Verarbeitungseinrichtung sich in unmittelbarer Nähe der Elektroden befinden, um Rauschabtasten und Mikrophonieeffekte zu verringern.

20. Vorrichtung nach Anspruch 15, 16, 17, 18 oder 19, wobei die Fläche der Elektroden behandelt ist, um elektrische Kontaktimpedanz zu verringern.

21. Vorrichtung nach Anspruch 15, 16, 17, 18, 19 oder 20, wobei die Verarbeitungseinrichtung einen Spannungsfolger umfasst, der eine hohe Eingangsimpedanz und einen niedrigen Vorspannungsstrom aufweist, um für die gemessenen Spannungen das Gleichtakt-Unterdrückungsverhältnis maximal zu machen.

22. Vorrichtung zum Diagnostizieren eines Gewebezustandes, aufweisend:
Eine Sonde mit einer Spitze;
zumindest ein Array von Elektroden auf der Spitze zum Erzeugen von Signalen in Reaktion auf einen Kontakt zwischen einer Sondenfläche und einer Probenfläche;
eine Signalerfassungs- und Verarbeitungseinrichtung zum Ermitteln des Zustand eines Gewebes, einschließlich der Zusammensetzung, der Struktur der Funktion, des Gesundheitszustandes bzw. Erkrankung des Gewebes unter Verwendung elektrischer Potentiale, die durch Drücken des getesteten Gewebes gegen eine Sondenfläche induziert werden, welche freiliegende Elektroden enthält, wobei das Ermitteln des Zustands des Gewebes das Analysieren der Signale umfasst, welche den zeitlichen Verlauf der Kontaktverteilung anzeigen, um die Amplitude und. Geschwindigkeit einer Verschiebung zu erfassen, die auf die Probenfläche im Verlauf der Zeit ausgeübt wird, und um die winkelmäßige Ausrichtung der Sondenfläche zu erfassen, und das interpretieren der Amplitude und Geschwindigkeit der Verschiebung und der winkelmäßigen Ausrichtung der Sondenfläche zum Ermitteln des Zustands des Gewebes.

23. Vorrichtung nach Anspruch 22, wobei die Elektroden aus Metall hergestellt sind, das aus der Gruppe ausgewählt ist, die aus Edelmetall, Silber, Gold, Palladium, Platin, Rhodium, Ruthenium, Iridium, Osmium, Kupfer, Aluminium und Wolfram oder einer Legierung aus mehreren Metallen besteht, die aus Silber/Silberchlorid, Edelstahl und Platin/Iridium oder einer beliebigen Kombination der vorstehend genannten Metalle ausgewählt sind.

24. Vorrichtung nach Anspruch 22 oder 23, wobei die Elektroden aus Metalldrähten hergestellt sind.

25. Vorrichtung nach Anspruch 22, 23 oder 24, wobei die Elektroden keinen Durchmesser aufweisen, der im Bereich von Sub-Mikrometer bis Millimeter liegt.

26. Vorrichtung nach Anspruch 22, 23, 24 oder 25, wobei die Verarbeitungseinrichtung sich in unmittelbarer Nähe zu den Elektroden befindet, um Rauschabtasten und Mikrophonieeffekte zu verringern.

27. Vorrichtung nach Anspruch 22, 23, 24, 25 oder 26, wobei die Fläche der Elektroden behandelt ist, um die elektrische Kontaktimpedanz zu verringern.

28. Vorrichtung nach Anspruch 22, 23, 24, 25, 26 oder 27 wobei die verarbeitungseinrichtung einen Spannungsfolger umfasst, der eine hohe Eingangsimpedanz und einen niedrigen Vorspannungsstrom aufweist, um für die gemessenen Spannungen das Gleichtakt-Unterdrückungsverhältnis maximal zu machen.

## Revendications

1. Procédé de caractérisation in vitro de l'évolution temporelle et spatiale du contact entre deux surfaces arbitraires au moyen d'ensembles d'électrodes, ledit procédé comprenant les étapes :
a) d'application d'une surface de sonde contenant au moins un ensemble d'électrodes contre une surface de spécimen, faisant générer des signaux par lesdites électrodes en réaction au contact avec ladite surface de spécimen ;
b) d'obtention des signaux en provenance desdites électrodes ; et
c) d'analyse desdits signaux indiquant l'évolution temporelle de la distribution du contact pour estimer l'amplitude et la vitesse d'un déplacement appliqué sur ladite surface de spécimen dans le temps et pour estimer l'orientation angulaire de ladite surface de sonde.

2. Procédé selon la revendication 1, dans lequel ladite surface de sonde n'est pas plane.

3. Procédé selon la revendication 1 ou 2, dans lequel l'ensemble d'électrodes comprend une pluralité d'ensembles qui s'étendent dans différentes dimensions.

4. Procédé selon la revendication 1, 2 ou 3, dans lequel l'ensemble d'électrodes est positionné sur la surface qui contient l'ensemble d'électrodes pour former des lignes, des cercles ou des formes curvilignes.

5. Procédé selon la revendication 1, 2, 3 ou 4, dans lequel les géométries de la surface du spécimen et de la surface qui contient l'ensemble d'électrodes ont une géométrie sélectionnée dans le groupe composé des surface plane totale ou partielle, surface révolutionnaire, surface irrégulière ou d'une combinaison de deux ou plusieurs surfaces.

6. Procédé selon la revendication 1, 2, 3, 4 ou 5, dans lequel l'ensemble d'électrodes comprend au moins trois électrodes, lesdites au moins trois électrodes définissant deux dimensions.

7. Procédé selon la revendication 1, 2, 3, 4, 5 ou 6, dans lequel un potentiel électrique est mesuré d'une façon différentielle comme la différence potentielle entre les électrodes adjacentes.

8. Procédé selon la revendication 1, 2, 3, 4, 5 ou 6, dans lequel un potentiel électrique est mesuré d'une façon absolue par rapport à une électrode fixe de l'ensemble ou une électrode de référence placée dans un bain de test et reliée à la terre.

9. Procédé selon la revendication 1, 2, 3, 4, 5, 6, 7 ou 8, dans lequel les signaux sont mesurés par un couplage capacitif avec des électrodes sans contact ayant des caractéristiques de bruit stables, une absence d'effet de corrosion et des propriétés d'isolation.

10. Utilisation du procédé selon l'une quelconque des revendications 1 à 9 pour indiquer au moins l'une de l'amplitude et de la vitesse du déplacement et l'orientation de la surface qui contient ledit au moins un ensemble d'électrodes par rapport à la surface de spécimen.

11. Utilisation du procédé selon l'une quelconque des revendications 1 à 9 pour caractériser la réaction mécanique ou la "conformité de la surface" d'une matière.

12. Procédé in vitro pour détecter l'état d'un tissu comprenant sa composition, sa structure, sa fonction, l'état de santé ou de maladie du tissu, en utilisant des potentiels électriques induits par la compression du tissu testé par rapport à une surface de sonde qui contient les électrodes exposées, ledit procédé comprenant les étapes :
a) de caractérisation de l'évolution temporelle et spatiale du contact entre deux surfaces arbitraires utilisant au moins un ensemble d'électrodes, ladite caractérisation de l'évolution temporelle et spatiale du contact entre deux surfaces arbitraires étant conforme au procédé défini dans l'une quelconque des revendications 1 à 9 ; et
b) d'interprétation de ladite évolution temporelle et spatiale du contact entre lesdites deux surfaces arbitraires pour déterminer l'état du tissu.

13. Procédé selon la revendication 12, dans lequel ladite surface de sonde n'est pas plane.

14. Procédé selon la revendication 12 ou 13, dans lequel le tissu est un cartilage.

15. Appareil pour caractériser l'évolution temporelle et spatiale du contact entre deux surfaces arbitraires, comprenant :
une sonde avec une extrémité ;
au moins un ensemble d'électrodes sur l'extrémité pour générer des signaux en réaction au contact entre une surface de sonde et une surface de spécimen ;
des moyens d'acquisition et de traitement de signal pour caractériser l'évolution temporelle et spatiale du contact entre deux surfaces arbitraires, ladite caractérisation comprenant l'analyse desdits signaux pour estimer l'amplitude et la vitesse d'un déplacement appliqué sur ladite surface de spécimen dans le temps et pour estimer l'orientation angulaire de ladite surface de sonde.

16. Appareil selon la revendication 15, dans lequel les électrodes sont constituées de métal sélectionné dans le groupe composé de métal noble, argent, or, palladium, platine, rhodium, ruthénium, iridium, osmium, cuivre, aluminium, et tungstène, ou d'un alliage de plusieurs métaux sélectionnés parmi l'argent/chlorure d'argent, acier inoxydable, et de platine/iridium ou de toute combinaison des métaux mentionnés ci-dessus.

17. Appareil selon la revendication 15 ou 16, dans lequel les électrodes sont composées de fils métalliques.

18. Appareil selon la revendication 15, 16 ou 17, dans lequel les électrodes ont un diamètre compris dans une plage allant d'une taille inférieure au micron au millimètre.

19. Appareil selon la revendication 15, 16, 17 ou 18, dans lequel les moyens de traitement sont à proximité étroite des électrodes pour réduire les effets de captage du bruit et microphoniques.

20. Appareil selon la revendication 15, 16, 17, 18 ou 19, dans lequel la surface des électrodes est traitée pour réduire l'impédance du contact électrique.

21. Appareil selon la revendication 15, 16, 17, 18, 19 ou 20, dans lequel le moyen de traitement comprend un suiveur de tension ayant une impédance d'entrée élevée et un courant de polarisation faible pour maximiser le rapport de réjection en mode commun pour les tensions mesurées.

22. Appareil pour diagnostiquer l'état d'un tissu, comprenant :
une sonde avec une extrémité ;
au moins un ensemble d'électrodes sur l'extrémité pour générer des signaux en réaction au contact entre une surface de sonde et une surface de spécimen ;
des moyens d'acquisition et de traitement de signal pour détecter l'état d'un tissu comprenant sa composition, sa structure, sa fonction, l'état de santé ou de maladie du tissu, en utilisant des potentiels électriques induits par la compression du tissu testé par rapport à une surface de sonde qui contient les électrodes exposées, ledit procédé comprenant l'analyse desdits signaux indiquant l'évolution temporelle de la distribution du contact pour estimer l'amplitude et la vitesse d'un déplacement appliqué sur ladite surface de spécimen dans le temps et pour estimer l'orientation angulaire de ladite surface de sonde, et interpréter ladite amplitude et ladite vitesse dudit déplacement et de ladite orientation angulaire de ladite surface de sonde pour déterminer l'état du tissu.

23. Appareil selon la revendication 22, dans lequel les électrodes sont constituées de métal sélectionné dans le groupe composé de métal noble, argent, or, palladium, platine, rhodium, ruthénium, iridium, osmium, cuivre, aluminium, et tungstène, ou d'un alliage de plusieurs métaux sélectionnés parmi l'argent/chlorure d'argent, acier inoxydable, et de platine/iridium ou de toute combinaison des métaux mentionnés ci-dessus.

24. Appareil selon la revendication 22 ou 23, dans leque.l les électrodes sont composées de fils métalliques.

25. Appareil selon la revendication 22, 23 ou 24, dans lequel les électrodes ont un diamètre compris dans une plage allant d'une taille inférieure au micron au millimètre.

26. Appareil selon la revendication 22, 23, 24 ou 25, dans lequel les moyens de traitement sont à proximité étroite des électrodes pour réduire les effets de captage du bruit et microphoniques.

27. Appareil selon la revendication 22, 23, 24, 25 ou 26, dans lequel la surface des électrodes est traitée pour réduire l'impédance du contact électrique.

28. Appareil selon la revendication 22, 23, 24, 25, 26 ou 27, dans lequel le moyen de traitement comprend un suiveur de tension ayant une impédance d'entrée élevée et un courant de polarisation faible pour maximiser le rapport de réjection en mode commun pour les tensions mesurées.
